(19) Europäisches Patentamt
European Patent Office
Office européen des brevets

(11) Numéro de publication : **0 467 748 A1**

(12) **DEMANDE DE BREVET EUROPEEN**

(21) Numéro de dépôt : **91401887.4**

(22) Date de dépôt : **08.07.91**

(51) Int. Cl.$^5$ : **G01N 24/10,** G01R 33/60

(30) Priorité : **10.07.90 FR 9008738**

(43) Date de publication de la demande : **22.01.92 Bulletin 92/04**

(84) Etats contractants désignés : **CH DE FR GB LI SE**

(71) Demandeur : **COMMISSARIAT A L'ENERGIE ATOMIQUE**
**31-33, rue de la Fédération**
**F-75015 Paris (FR)**

(72) Inventeur : **Moussavi, Mehdi**
**20 rue Joseph Rolland**
**F-38120 Saint Egreve (FR)**

(74) Mandataire : **Mongrédien, André et al**
**c/o BREVATOME 25, rue de Ponthieu**
**F-75008 Paris (FR)**

(54) **Procédé de détection ou de dosage d'oxygène par spectrométrie rpe utilisant des phtalocyanines de lithium radicalaire et composition utilisable pour le dosage "in Vivo".**

(57) L'invention a pour objet un procédé de détection ou de dosage d'oxygène moléculaire par spectrométrie RPE utilisant des phtalocyanines de lithium radicalaire et composition utilisable pour le dosage "in vivo".

Ce procédé consiste à

— mettre en contact avec ledit milieu une phtalocyanine de lithium radicalaire répondant à la formule :

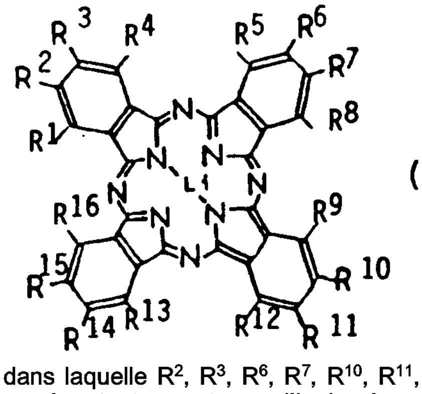

(I)

dans laquelle $R^2$, $R^3$, $R^6$, $R^7$, $R^{10}$, $R^{11}$, $R^{14}$ et $R^{15}$ représentent un atome d'hydrogène, un atome de deutérium ou un radical alkyle ou alcoxy en $C_1$ à $C_3$, et $R^1$, $R^4$, $R^5$, $R^8$, $R^9$, $R^{12}$, $R^{13}$ et $R^{16}$ représentent un atome d'hydrogène ou de deutérium, à condition que $R^1$ à $R^{16}$ ne représentent pas tous un atome d'hydrogène, et

— examiner le signal RPE de la phtalocyanine de lithium radicalaire en contact avec ledit milieu.

EP 0 467 748 A1

La présente invention a pour objet un procédé de dosage in vitro ou in vivo d'oxygène moléculaire dans un milieu, utilisable notamment pour le dosage "in vivo" de l'oxygène intracellulaire.

Le dosage d'oxygène moléculaire présent dans certains organes du corps humain est un moyen d'investigation intéressant car il permet de détecter les cellules malades de l'organe qui ont des teneurs en oxygène inférieures à la normale.

Récemment, on a trouvé que la mesure de la concentration en oxygène moléculaire pouvait être effectuée par résonance paramagnétique électronique (RPE). Dans cette technique, on utilise un traceur radicalaire dont les caractéristiques RPE telles que la largeur de raie, varient notablement en fonction de la teneur en oxygène du milieu dans lequel il se trouve. Des techniques de dosage de l'oxygène moléculaire par spectrométrie RPE sont décrites par exemple dans les documents suivants :
Swartz, Pure & Appl. Chem., vol. 62, n° 2, p. 235-239, 1990 ; Woods et al, Journal of Magnetic Resonance, n° 85, p. 50-59, 1989.

Pour mesurer la concentration d'oxygène intracellulaire par cette technique, on injecte ou on implante dans le milieu ou l'organe dont on veut connaître la teneur en oxygène, une substance radicalaire, puis on examine cette substance pendant qu'elle est dans ce milieu au moyen d'un spectromètre à résonance paramagnétique électronique. A partir de la largeur de raie RPE obtenue, on en déduit la teneur en oxygène du milieu en se référant à une courbe d'étalonnage préalable d'un échantillon de la substance radicalaire donnant la relation entre la largeur de raie RPE et la teneur en oxygène.

Les substances radicalaires utilisées dans de tels procédés doivent présenter différentes propriétés. Ainsi, elles doivent être biocompatibles et avoir des caractéristiques RPE qui changent notablement en fonction de la teneur en oxygène du milieu, au moins dans la gamme des concentrations à suivre, qui est de 0 à 10% d'oxygène moléculaire dans le cas des organes humains qu'il est intéressant d'examiner.

Parmi les substances radicalaires utilisables dans de tels procédés, on a déjà envisagé l'utilisation du 2,2,6,6-tétraméthylpyridine-N-oxyl-4-one (TANO), mais celui-ci a l'inconvénient de ne présenter qu'une faible variation de ses caractéristiques RPE dans la gamme de concentrations en oxygène la plus intéressante (0 à 10%).

La présente invention a précisément pour objet un procédé de dosage ou de détection de l'oxygène moléculaire dans un milieu, qui utilise des substances radicalaires ayant une meilleure sensibilité que le TANO.

Selon l'invention, le procédé de détection ou de dosage d'oxygène moléculaire dans un milieu consiste à :

– mettre en contact avec ledit milieu une phtalocyanine de lithium radicalaire répondant à la formule :

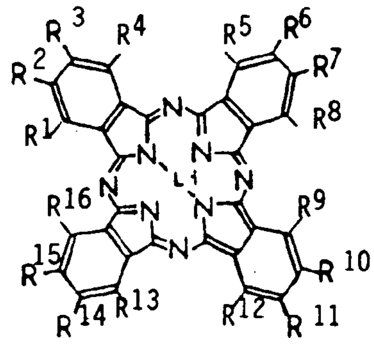

(I)

dans laquelle $R^2$, $R^3$, $R^6$, $R^7$, $R^{10}$, $R^{11}$, $R^{14}$ et $R^{15}$ qui peuvent être identiques ou différents, représentent un atome d'hydrogène, un atome de deutérium ou un radical alkyle ou alcoxy en $C_1$ à $C_3$, et $R^1$, $R^4$, $R^5$, $R^8$, $R^9$, $R^{12}$, $R^{13}$ et $R^{16}$ qui peuvent être identiques ou différents, représentent un atome d'hydrogène ou de deutérium, à condition que $R^1$ à $R^{16}$ ne représentent pas tous un atome d'hydrogène, et

– examiner le signal RPE de la phtalocyanine de lithium radicalaire en contact avec ledit milieu.

Dans ce procédé, l'utilisation d'une phtalocyanine de lithium radicalaire répondant à la formule (I) précitée permet d'obtenir une meilleure sensibilité et une meilleure précision sur la mesure des faibles taux d'oxygène, en particulier grâce à la présence des substituants alkyle ou alkoxy, ou des atomes de deutérium.

En effet, la substitution en périphérie de la phtalocyanine par des radicaux alkyle ou alcoxy a pour effet d'obturer en partie les canaux à oxygène. De ce fait, la diffusion d'oxygène se fait plus entre les couches de phtalocyanine que dans les colonnes de phtalocyanine et il en résulte une réponse plus rapide à l'oxygène, c'est-à-dire un élargissement de la raie RPE des phtalocyanines substituées beaucoup plus rapide.

Ce résultat n'était pas prévisible à partir des performances que l'on obtient avec la phtalocyanine de lithium radicalaire connue qui présentait une largeur de raie RPE trop importante (30 000nT) et très peu de variation de la largeur de raie lorsqu'on augmente la concentration en oxygène du milieu dans lequel se trouve la phtalocyanine radicalaire de lithium connue.

Selon un premier mode de réalisation du procédé de l'invention, la phtalocyanine de lithium radicalaire répondant à la formule (I) précitée est une phtalocyanine de lithium octasubstituée par un radical alkyle ou alkoxy. Les radicaux alkyle ou alkoxy peuvent être en particulier le radical méthyle ou méthoxy et ils se trouvent de préférence dans les positions $R^2$, $R^3$, $R^6$, $R^7$, $R^{10}$, $R^{11}$, $R^{14}$ et $R^{15}$, les autres positions $R^1$, $R^4$, $R^5$, $R^8$

$R^9$, $R^{12}$, $R^{13}$ et $R^{16}$ étant occupées par des atomes d'hydrogène.

Des phtalocyanines de lithium octasubstituées de ce type sont décrites en particulier dans le document EP-A- 0 352 182, et elles peuvent être préparées par oxydation monoélectronique par voie galvanostatique de la phtalocyanine octasubstituée de dilithium correspondante comme il est décrit dans le document EP-A-0 352 182.

Selon un second mode de réalisation du procédé de l'invention, on utilise une phtalocyanine de lithium radicalaire octasubstituée et au moins partiellement deutérée.

Dans ce cas, la substitution est de préférence effectuée par un radical méthyle ou méthoxy sur les positions $R^2$, $R^3$, $R^6$, $R^7$, $R^{10}$, $R^{11}$, $R^{14}$ et $R^{15}$ de la formule (I) précitée, l'une au moins des autres positions $R^1$; $R^4$, $R^5$, $R^8$, $R^9$, $R^{12}$, $R^{13}$ et $R^{16}$ étant occupée un atome de deutérium et le reste de ces dernières positions étant occupé par des atomes d'hydrogène.

Le fait de deutérer la phtalocyanine de lithium radicalaire octasubstituée permet encore d'améliorer les performances de cette substance radicalaire pour le dosage de l'oxygène par spectrométrie RPE.

Ces phtalocyanines de lithium octasubstituées et deutérées peuvent être préparées également par oxydation monoélectronique du précurseur dilithié et deutéré correspondant. Ce précurseur dilithié deutéré peut être obtenu par un procédé d'échange isotopique HD entre la phtalocyanine octasubstituée et dilithiée et de la tétraméthylpyridine deutérée, en présence d'un catalyseur à base de tétraméthylpyridine deutérée et lithiée.

Selon un troisième mode de réalisation du procédé de l'invention, la substance radicalaire utilisée est une phtalocyanine de lithium radicalaire deutérée au moins en partie. Dans ce cas, l'un au moins des $R^1$ à $R^{16}$ est un atome de deutérium et les autres $R^1$ à $R^{16}$ représentent un atome d'hydrogène. De préférence, les $R^1$ à $R^{16}$ sont tous des atomes de deutérium car ceci permet d'améliorer de façon plus importante les caractéristiques RPE de la phtalocyanine de lithium radicalaire et de lui conférer une meilleure sensibilité à l'oxygène.

Cette phtalocyanine de lithium radicalaire deutérée au moins partiellement peut être préparée par oxydation monoélectronique du précurseur dilithié deutéré. Ce précurseur dilithié deutéré peut être préparé par échange isotopique H-D comme dans le cas des phtalocyanines de lithium radicalaire octasubstituées et deutérées.

Les phtalocyanines radicalaires de formule (I) utilisées dans le procédé de l'invention sont très intéressantes car la largeur de leur raie RPE varie pratiquement de façon linéaire avec la teneur en oxygène, dans la gamme des concentrations en oxygène allant de 0 à 10%.

Grâce à ces caractéristiques, on peut obtenir une meilleure précision sur la mesure des faibles taux d'oxygène dans les tissus biologiques. De plus, la forte sensibilité de ces phtalocyanines à l'oxygène permet, soit d'injecter des quantités plus faibles de substance radicalaire si cela s'avère nécessaire, soit de diminuer le temps d'acquisition pour les courbes RPE et obtenir ainsi une réponse plus rapide sur les métabolismes d'organes.

Pour les dosages d'oxygène "in vivo", par exemple dans le coeur ou dans d'autres organes, on peut implanter un cristal de la phtalocyanine radicalaire de formule (I) directement dans l'organe à examiner, ou utiliser une suspension dans un liquide de poudre de phtalocyanine radicalaire de formule (I) que l'on injecte au patient à examiner.

Les liquides utilisés pour préparer de telles suspensions sont des liquides biocompatibles et non toxiques.

A titre d'exemple, on peut utiliser des alcools en $C_4$ à $C_{10}$ comme l'hexanol, des polyalcools en $C_4$ à $C_{10}$, de l'alcool furfurylique ou du vératrol.

La suspension peut comprendre de plus si nécessaire d'autres additifs tels que des agents stabilisant la suspension ou réglant sa viscosité à une valeur appropriée.

La concentration en phtalocyanine radicalaire de la suspension peut varier dans un large intervalle ; de préférence, elle se situe dans la gamme allant de 0,1 à 10mg/l.

Bien que les phtalocyanines de lithium radicalaire utilisées dans le procédé de l'invention ne soient pas toxiques, les doses administrées sont généralement faibles et peuvent aller par exemple de 0,01 à 0,1mg/kg de poids corporel.

Après implantation des cristaux ou administration de la suspension, on peut réaliser l'examen par spectrométrie RPE rapidement.

Une méthode utilisant la spectrométrie RPE pour doser l'oxygène à l'aide d'un marqueur du type nitroxyde comme le TANO, a été décrite dans Journal of Magnetic Resonance, n° 85, p. 50-59, 1989, mais elle utilise un spectromètre RPE à 9GHz.

Avec les phtalocyanine de l'invention, on peut effectuer des mesures non seulement à 9GHz mais aussi à 250MHz. L'utilisation de la basse fréquence est préférable pour l'oxymétrie in vivo ou in vitro dans des solvants polaires tels que l'eau.

En effet, la pénétration du champ magnétique de polarisation à 9-10GHz (environ 3000 Gauss) n'est que de quelques millimètres alors qu'à 250MHz, on peut envisager une pénétration, donc des mesures possibles, sur plusieurs centimètres (environ 15-20cm).

Si on devait utiliser les nitroxydes pour l'oxymétrie à basse fréquence, on serait obligé d'augmenter la quantité utilisée pour des raisons de sensibilité car la sensibilité en RPE chute avec le carré de la fréquence .

Ainsi, pour passer de 10GHz à 250MHz,(rapport de 40),et obtenir une sensibilité équivalente, il faudrait utiliser 40x40=1600 fois plus de nitroxyde alors que ce marqueur est connu pour être toxique à des doses à peine plus élevées que celles utilisées à 9GHz.

En revanche, ceci est possible avec les phtalocyanines de l'invention car celles-ci sont beaucoup plus sensibles et les quantités à utiliser sont de ce fait inférieures.

D'autres caractéristiques et avantages de l'invention apparaîtront mieux à la lecture des exemples qui suivent, donnés bien entendu à titre illustratif et non limitatif en référence au dessin annexé sur lequel :

– la figure 1 représente de façon schématique un dispositif convenant à la réalisation de la courbe d'étalonnage de la substance radicalaire utilisée dans le procédé de l'invention,

– la figure 2 est un diagramme représentant les variations de la largeur de raie RPE en fonction de la teneur en oxygène d'un milieu pour les phtalocyanines de lithium radicalaire octasubstituées ($(CH_3)_8PcLi$ et $(CH_3O)_8PcLi$) et pour une substance radicalaire utilisée dans l'art antérieur (TANO),

– la figure 3 représente de façon schématique une cellule d'électrolyse pour la préparation de phtalocyanine de lithium radicalaire deutérée,

– la figure 4 est un diagramme représentant le signal RPE obtenu avec la phtalocyanine de lithium radicalaire deutérée, et

– la figure 5 est un diagramme représentant le signal RPE obtenu avec la phtalocyanine de lithium octasubstituée ($(CH_3)_8(PcLi:H)$ et la phtalocyanine de lithium octasubstituée et deutérée en partie ($(CH_3)_8PcLi:D$).

## Exemple 1.

Cet exemple concerne l'utilisation d'octaméthyl phtalocyanine de lithium radicalaire pour la mesure de concentrations en oxygène allant de 0 à 10%.

On prépare l'octaméthyl phtalocyanine de lithium radicalaire à partir de l'octaméthyl phtalocyanine de dilithium en utilisant le même mode opératoire que dans l'exemple 2 de EP-A- 0 352 182.

On détermine ensuite les caractéristiques RPE de l'octaméthyl phtalocyanine de lithium radicalaire en présence de concentrations d'oxygène différentes pour obtenir la courbe d'étalonnage correspondant aux variations de la largeur de raie RPE en fonction de la teneur en oxygène du milieu.

Dans ce but, on introduit dans un tube les cristaux de phtalocyanine de lithium radicalaire octasubstituée obtenus précédemment, puis on fait le vide dans le tube et on mesure la largeur de la raie RPE après avoir introduit le tube maintenu sous vide dans un spectromètre RPE.

Ensuite, on refait les mêmes mesures après avoir mis en contact les cristaux avec des quantités différentes d'oxygène.

Ceci peut être effectué en utilisant le dispositif représenté de façon schématique sur la figure 1. Ce dispositif comprend une rampe à vide (11) munie d'un groupe de pompage (13) pour faire le vide dans la rampe qui peut être raccordée par l'intermédiaire d'une vanne (15) avec un tube (17) contenant les cristaux de phtalocyanine de lithium et par une vanne (19) avec un récipient (21) contenant un volume donné d'oxygène.

Pour effectuer la première mesure, on ouvre la vanne (15) après avoir disposé le tube (17) sur l'appareil et on fait le vide avec le groupe de pompage (13), puis on referme la vanne (15) et l'on introduit le tube (17) obturé dans le spectromètre RPE. Après cette mesure, on dispose à nouveau le tube (17) sur la rampe à vide (11), puis on ouvre la vanne (15) et la vanne (19) pour mettre en contact la phtalocyanine de lithium avec le volume donné d'oxygène du récipient (21). Après cette mise en contact, on ferme la vanne (15) et on introduit le tube (17) dans le spectromètre RPE pour avoir la largeur de raie à 9GHz.

Après cette opération, on dégaze à nouveau la phtalocyanine de lithium en disposant le tube (17) sur la rampe à vide (11) et en faisant le vide dans l'installation, puis on met ensuite en contact la phtalocyanine dégazée avec un autre volume d'oxygène en utilisant un autre récipient (21). On obtient ainsi les valeurs de la largeur de raie RPE de cette phtalocyanine en fonction de la concentration en oxygène.

Sur la figure 2, on a représenté la courbe de variations de la largeur de raie RPE (en $10^{-7}$T) de $(CH_3)_8PcLi$ en fonction de la teneur en oxygène (en %).

Sur cette figure, on voit que la variation de la largeur de raie est linéaire dans le domaine de concentrations allant de 0 à 10% et que l'on peut détecter des variations très faibles de la teneur en oxygène dans cette gamme de concentrations.

Sur cette figure, on a représenté à titre comparatif les variations de la largeur de raie RPE obtenues dans les mêmes conditions avec le TANO qui était précédemment utilisé pour le dosage d'oxygène par RPE.

Si l'on compare ces deux courbes, on remarque que le TANO ne permet pas de détecter des variations de concentration d'oxygène dans le domaine allant de 0 à 6%, ce qui montre bien l'intérêt de l'invention.

## Exemple 2.

Dans cet exemple, on suit le même mode opératoire que dans l'exemple 1 pour déterminer les variations de la largeur de raie RPE de l'octaméthoxy phtalocyanine de lithium radicalaire préparée en suivant le mode opératoire décrit dans l'exemple 1 du document EP-A- 0 352 182.

Les résultats obtenus sont donnés sur la figure 2

pour cette phtalocyanine de lithium $(CH_3O)_8PcLi$.

Sur cette figure, on remarque que la variation de la largeur de la raie RPE de $(CH_3O)_8PcLi$ est également linéaire dans le domaine de concentrations allant de 0 à 10% et que la sensibilité est meilleure que celle de $(CH_3)_8PcLi$.

Ainsi, cette phtalocyanine de lithium radicalaire octasubstituée présente aussi des performances beaucoup plus intéressantes que le TANO puisqu'elle permet de détecter de faibles variations d'oxygène dans la gamme de concentrations allant de 0 à 10%.

**Exemple 3** : Préparation de phtalocyanine de lithium deutérée.

Dans cet exemple, on prépare la phtalocyanine de lithium radicalaire deutérée à partir de son précurseur dilithié deutéré.

a) Préparation de la phtalocyanine dilithiée deutérée.

Pour cette préparation, on met en contact de la phtalocyanine de lithium dilithiée avec de la ($^2$H)-2,2,6,6-tétraméthylpipéridine.

La ($^2$H)-2,2,6,6-tétraméthylpipéridine a été préparée par échange isotopique H-D entre la tétraméthylpyridine (TMP) du commerce et $^2H_2O$ à la température ambiante, suivi d'une séparation de la phase organique et de la phase aqueuse par addition d'éther.

On réalise l'échange entre la phtalocyanine dilithiée et la TMP deutérée, en présence de tétraméthylpipéridine lithium qui joue le rôle de catalyseur, en mélangeant tous ces réactifs dans du tétrahydrofuranne à -23°C.

Dans ces conditions, l'échange isotopique se fait sélectivement sur les positions correspondant aux $R^1$, $R^4$, $R^5$, $R^8$, $R^9$, $R^{12}$, $R^{13}$ et $R^{16}$ de la formule (I). On répète donc l'échange plusieurs fois pour obtenir la phtalocyanine de lithium de formule (I) entièrement deutérée, c'est-à-dire dans laquelle les $R^1$ à $R^{16}$ sont des atomes de deutérium.

On suit l'échange de deutérium par résonance magnétique nucléaire du proton car le remplacement des atomes d'hydrogène par le deutérium dans les positions qui correspondent aux $R^1$, $R^4$, $R^5$, $R^8$, $R^9$, $R^{12}$, $R_{13}$ et $R^{16}$ se traduit par l'apparition de singulets sur le spectre de résonance magnétique nucléaire du proton alors que dans le cas où l'on a deux atomes d'hydrogène dans les positions adjacentes $R^1$-$R^2$ ou $R^3$-$R^4$ etc. le couplage entre les protons donne un signal RMN constitué de deux multiplets (quadruplets) à 8 et 9,2ppm.

b) Préparation de la phtalocyanine de lithium radicalaire deutérée.

Pour cette préparation, on utilise la cellule d'électrolyse représentée sur la figure 3.

Sur cette figure, on a représenté de façon schématique une cellule d'électrolyse utilisable pour préparer la poudre de phtalocyanine de lithium radicalaire deutérée de l'invention. Cette cellule (1) a une forme semisphérique et elle est fermée de façon étanche par un couvercle (3). A l'intérieur de la cellule, sont disposées une cathode (5)constituée par un fil métallique de platine ayant par exemple un diamètre de 1mm et une longueur de 4cm, et une anode (7) constituée par une plaque de platine de 25x35mm. L'anode et la cathode sont reliées à un générateur de courant électrique muni d'un dispositif approprié permettant de faire fonctionner la cellule selon le mode intensiostatique ou galvanostatique.

On introduit dans la cellule (1) 300mg de la phtalocyanine dilithiée deutérée obtenue précédemment en solution dans 500ml d'acétonitrile de qualité ultra anhydre. L'acétonitrile utilisé a une teneur en eau inférieure à 50ppm et il a été passé juste avant l'utilisation sur une colonne de poudre d'alumine basique d'activité I ayant un diamètre de 5cm et une hauteur de 10cm, pour éliminer les traces d'acide acétique qu'il contient éventuellement. On introduit également dans la cellule 400mg d'un électrolyte-support constitué par de l'hexafluorophosphate de tétrabutylammonium qui a été recristallisé 5 fois dans l'éthanol et séché pendant 24h sous vide à 80°C.

Après avoir fermé la cellule d'électrolyse, on réalise l'électrosynthèse en utilisant un courant constant de 5μA pendant 24h, puis un courant constant de 20μA pendant 48h et un courant constant de 50μA pendant 72h.

On recueille ainsi en fin d'opération, 150mg de phtalocyanine de lithium radicalaire deutérée.

Cette phtalocyanine de lithium radicalaire deutérée présente sensiblement les mêmes performances que $(CH_3)_8PcLi$ pour le dosage de l'oxygène.

Sur la figure 4, on a représenté le signal RPE obtenu à 9GHz avec la phtalocyanine de lithium partiellement deutérée (PcLi : D) préparée précédemment. Sur cette figure on a également représenté en pointillés le signal obtenu dans les mêmes conditions avec de la phtalocyanine de lithium de l'art antérieur préparée par oxydation monoélectronique par voie potentiostatique à +0,5V du précurseur dilithié, comme cela est décrit par Sugimoto et al dans J. Chem. Soc., Chem. Commun., 1986, p. 962 et 963.

Sur cette figure, on remarque que le signal obtenu avec la phtalocyanine de lithium partiellement deutérée a une pente plus forte donc une meilleure sensibilité que le signal obtenu avec la phtalocyanine de lithium non deutérée.

En effet, plus le signal obtenu est fin, plus les faibles variations de largeur de raie RPE sont détectables, plus les quantités d'oxygène détectées sont faibles.

**Exemple 4.**

Dans cet exemple on prépare de la même façon que dans l'exemple 2 du EP-A- 0 352 182 de l'octaméthyl phtalocyanine de lithium deutérée en partant de son précurseur dilithié, deutéré, préparé de la même façon que dans l'exemple 3 ci-dessus.

On examine ensuite dans un spectromètre RPE la phtalocyanine de lithium octasubstituée par des radicaux méthyle et partiellement deutérée sur les positions qui correspondent aux $R^1$, $R^4$, $R^5$, $R^8$, $R^9$, $R^{12}$, $R^{13}$ et $R^{16}$ de la formule (I).

Les résultats obtenus sont donnés sur la figure 5 qui représente le signal RPE de cette phtalocyanine $(CH_3)_8PcLi{:}D$.

Sur cette figure, on a également représenté le signal RPE de la phtalocyanine octaméthyl substituée, non deutérée $(CH_3)_8PcLi$, obtenu dans les mêmes conditions.

On remarque ainsi que la substitution par le deutérium permet d'obtenir de meilleures caractéristiques RPE puisque la pente du signal est plus forte dans le cas de $(CH_3)_8PcLi{:}D$.

## Revendications

1. Procédé de détection ou de dosage d'oxygène moléculaire dans un milieu, caractérisé en ce qu'il consiste à
   – mettre en contact avec ledit milieu une phtalocyanine de lithium radicalaire répondant à la formule :

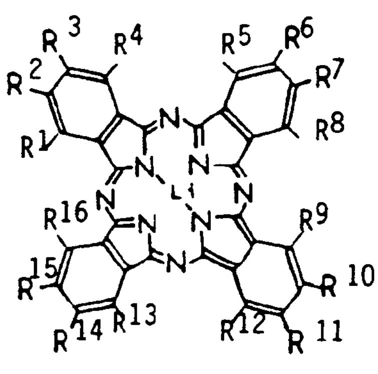

(I)

   dans laquelle $R^2$, $R^3$, $R^6$, $R^7$, $R^{10}$, $R^{11}$, $R^{14}$ et $R^{15}$ qui peuvent être identiques ou différents, représentent un atome d'hydrogène, un atome de deutérium ou un radical alkyle ou alcoxy en $C_1$ à $C_3$, et $R^1$, $R^4$, $R^5$, $R^8$, $R^9$, $R^{12}$, $R^{13}$ et $R^{16}$ qui peuvent être identiques ou différents, représentent un atome d'hydrogène ou de deutérium, à condition que $R^1$ à $R^{16}$ ne représentent pas tous un atome d'hydrogène, et
   – examiner le signal RPE de la phtalocyanine de lithium radicalaire en contact avec ledit milieu.

2. Procédé selon la revendication 1, caracterisé en ce que $R^2$, $R^3$, $R^6$, $R^7$, $R^{10}$, $R^{11}$, $R^{14}$ et $R^{15}$ représentent le radical méthyle et $R^1$, $R^4$, $R^5$, $R^8$, $R^9$, $R^{12}$, $R^{13}$ et $R^{16}$ représentent un atome d'hydrogène.

3. Procédé selon la revendication 1, caractérisé en ce que $R^2$, $R^3$, $R^6$, $R^7$, $R^{10}$, $R^{11}$, $R^{14}$ et $R^{15}$ représentent le radical méthoxy et $R^1$, $R^4$, $R^5$, $R^8$, $R^9$, $R^{12}$, $R^{13}$ et $R^{16}$ représentent un atome d'hydrogène.

4. Procédé selon la revendication 1, caractérisé en ce que $R^2$, $R^3$, $R^6$, $R^7$, $R^{10}$, $R^{11}$, $R^{14}$ et $R^{15}$ représentent le radical méthyle ou méthoxy et l'un au moins des $R^1$, $R^4$, $R^5$, $R^8$, $R^9$, $R^{12}$, $R^{13}$ et $R^{16}$ représente un atome de deutérium, le reste des $R^1$, $R^4$, $R^5$, $R^8$, $R^9$, $R^{12}$, $R^{13}$ et $R^{16}$ étant un atome d'hydrogène.

5. Procédé selon la revendication 1, caracterisé en ce que l'un au moins des $R^1$ à $R^{16}$ représente un atome de deutérium et les autres $R^1$ à $R^{16}$ représentent un atome d'hydrogène.

6. Composition utilisable pour la détection ou le dosage d'oxygène moléculaire, caractérisée en ce qu'elle comprend une phtalocyanine de lithium radicalaire répondant à la formule :

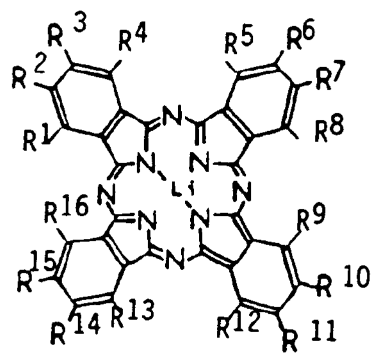

(I)

   dans laquelle $R^2$, $R^3$, $R^6$, $R^7$, $R^{10}$, $R^{11}$, $R^{14}$ et $R^{15}$ qui peuvent être identiques ou différents, représentent un atome d'hydrogène, un atome de deutérium ou un radical alkyle ou alcoxy de 1 à 3 atomes de carbone, et les $R^1$, $R^4$, $R^5$, $R^8$, $R^9$, $R^{12}$, $R^{13}$ et $R^{16}$ qui peuvent être identiques ou différents, représentent un atome d'hydrogène ou de deutérium à condition que $R^1$ à $R^{16}$ ne représentent pas tous un atome d'hydrogène.

7. Composition selon la revendication 6, caractérisée en ce qu'elle est constituée par une suspension d'une poudre de la phtalocyanine de lithium radicalaire de formule (I) dans un liquide biocompatible et non toxique.

**8.** Composition selon la revendication 7, caractérisée en ce que le liquide est choisi parmi les alcools en $C_4$ à $C_{10}$, l'alcool furfurylique, les polyalcools en $C_4$ à $C_{10}$ et le vératrol.

**9.** Composition selon l'une quelconque des revendications 6 à 8, caractérisée en ce que $R^2$, $R^3$, $R^6$, $R^7$, $R^{10}$, $R^{11}$, $R^{14}$ et $R^{15}$ représentent le radical méthyle et $R^1$, $R^4$, $R^5$, $R^8$, $R^9$, $R^{12}$, $R^{13}$ et $R^{16}$ représentent un atome d'hydrogène.

**10.** Composition selon l'une quelconque des revendications 6 à 9, caractérisée en ce que $R^2$, $R^3$, $R^6$, $R^7$, $R^{10}$, $R^{11}$, $R^{14}$ et $R^{15}$ représentent le radical méthoxy et $R^1$, $R^4$, $R^5$, $R^8$, $R^9$, $R^{12}$, $R^{13}$ et $R^{16}$ représentent un atome d'hydrogène.

**11.** Composition selon l'une quelconque des revendications 6 à 9, caractérisée en ce que $R^2$, $R^3$, $R^6$, $R^7$, $R^{10}$, '$R^{11}$, $R^{14}$ et $R^{15}$ représentent le radical méthyle ou méthoxy et l'un au moins des $R^1$, $R^4$, $R^5$, $R^8$, $R^9$, $R^{12}$, $R^{13}$ et $R^{16}$ représente un atome de deutérium, le reste des $R^1$, $R^4$, $R^5$, $R^8$, $R^9$, $R^{12}$, $R^{13}$ et $R^{16}$ étant un atome d'hydrogène.

**12.** Composition selon l'une quelconque des revendications 6 à 9, caracterisée en ce que l'un au moins des $R^1$ à $R^{16}$ représente un atome de deutérium et les autres $R^1$ à $R^{16}$ représentent un atome d'hydrogène.

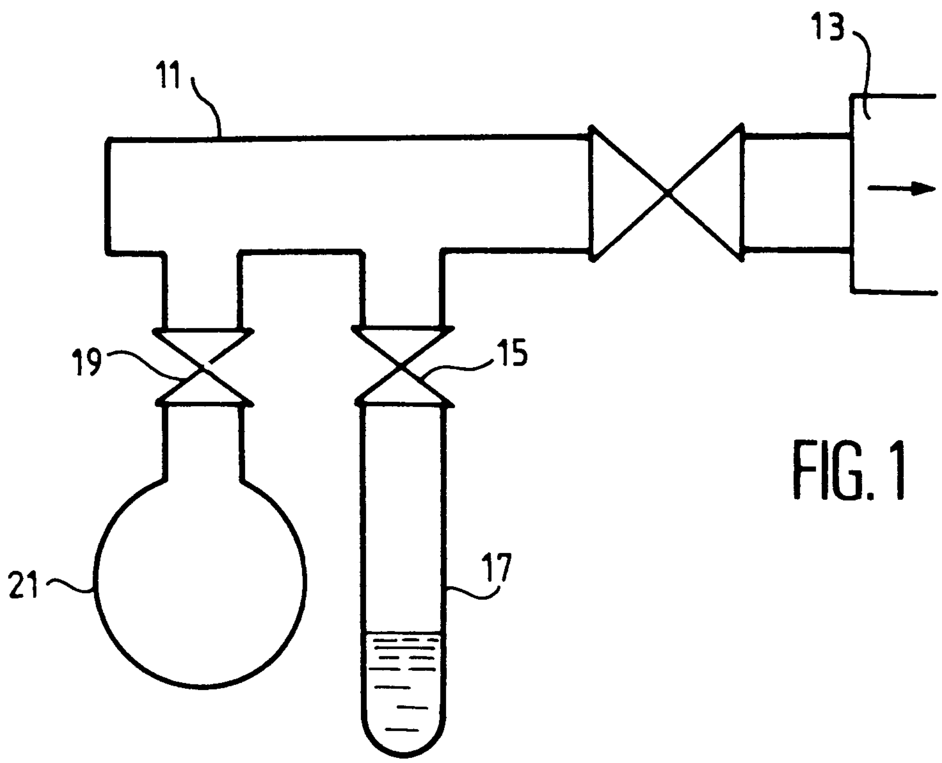


FIG. 1

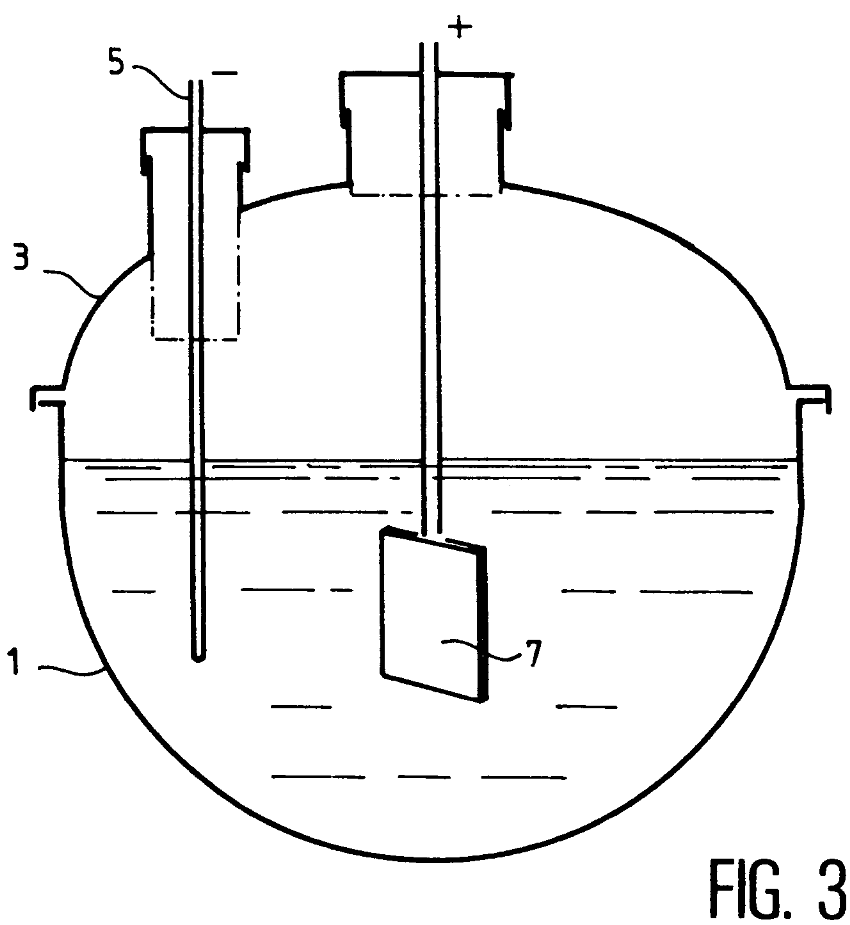


FIG. 3

FIG. 2

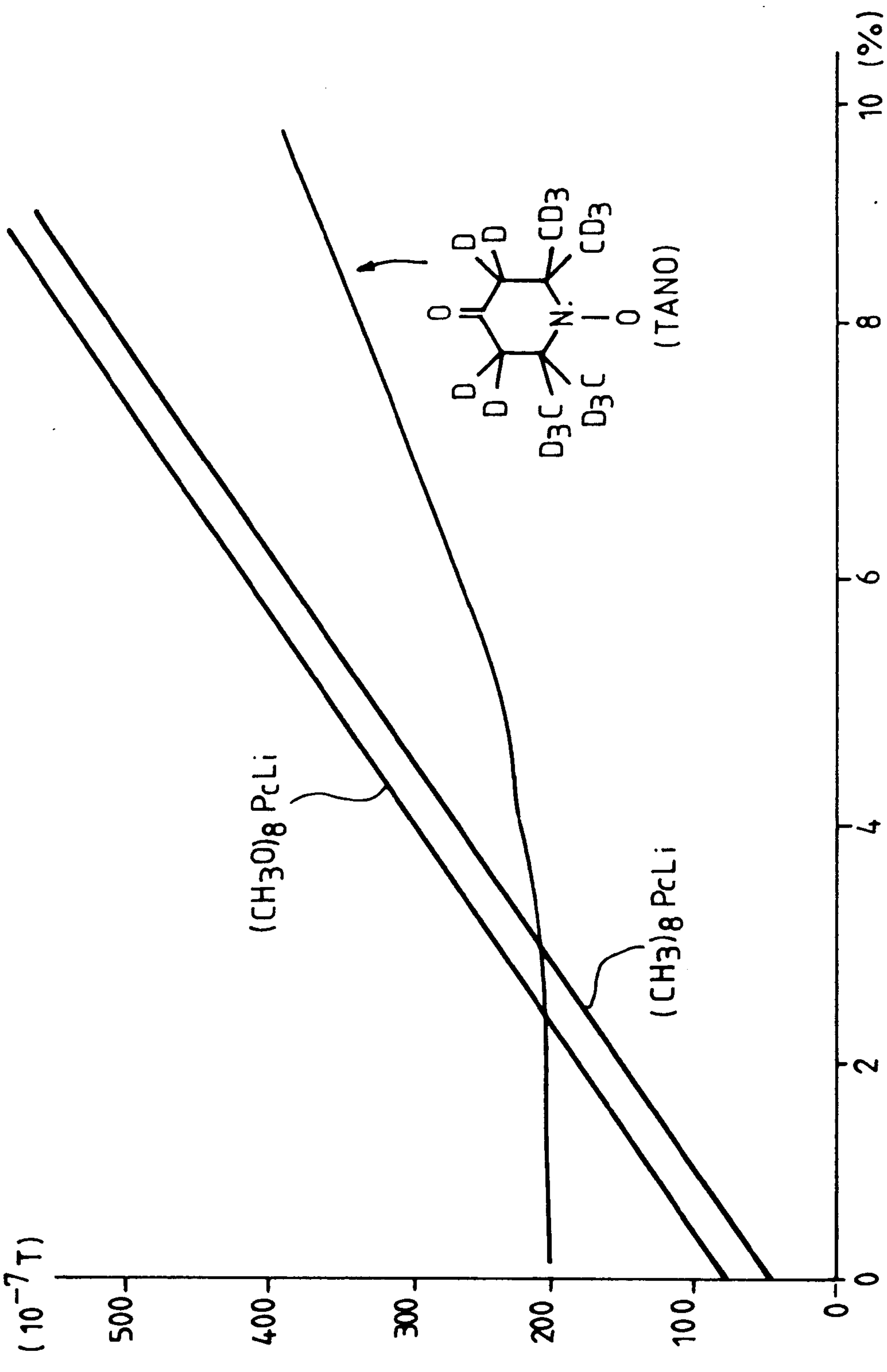

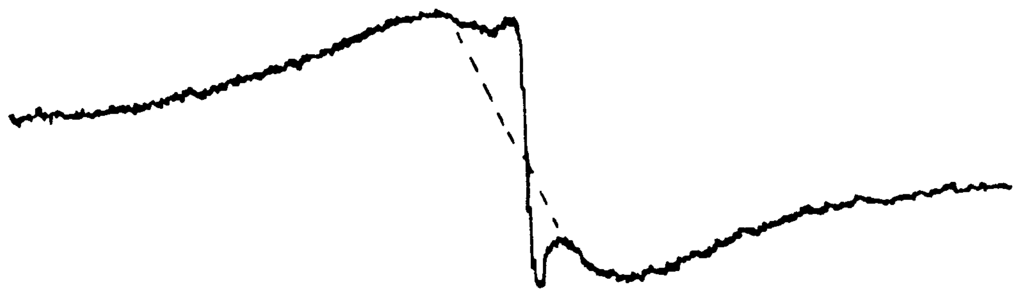

FIG. 4

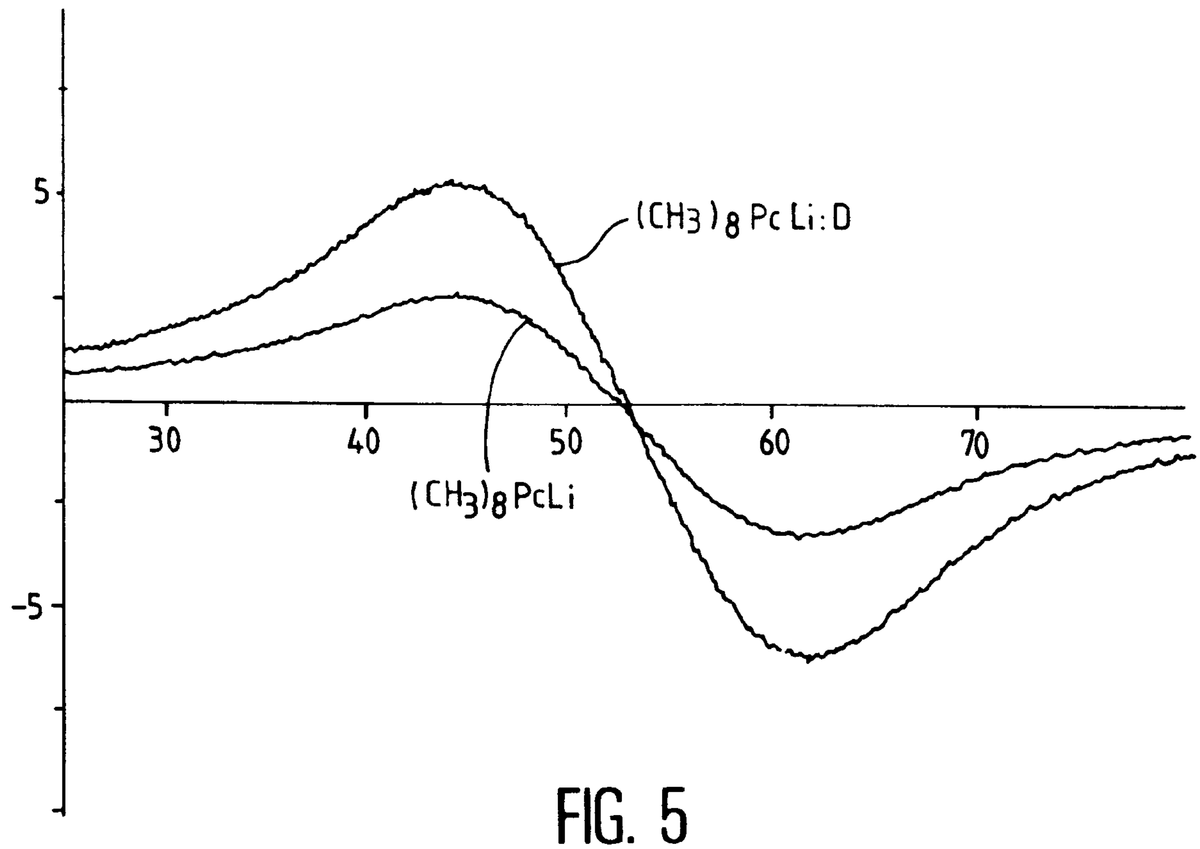


FIG. 5

Office européen des brevets

# RAPPORT DE RECHERCHE EUROPEENNE

Numero de la demande

EP 91 40 1887

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | SOLID STATE COMMUNICATIONS, vol. 63, no. 8, 1987, pages 741-744, Oxford, GB; P. TUREK et al.: "Extreme spin exchange narrowing in a neutral phthalocyanine radical: the lithium phthalocyanine [1]<br>* En entier *<br>--- | 1,6 | G 01 N 24/10<br>G 01 R 33/60 |
| A | MOLECULAR CRYSTALS AND LIQUID CRYSTALS, vol 176, 1989, pages 535-546, Montreaux, CH; P. TUREK et al.: "SEPTET spin state in the lithium phthalocyanine pi-radical compound. Role of dioxygen"<br>* En entier *<br>--- | 1,6 | |
| A,D | JOURNAL OF MAGNETIC RESONANCE, vol. 85, no. 1, 15 octobre 1989, pages 50-59, Duluth, MN, US; R.K. WOODS et al.: "Spectral-spatial ESR imaging as a method of noninvasive biological oximetry"<br>* En entier *<br>--- | 1,6 | **DOMAINES TECHNIQUES RECHERCHES (Int. Cl.5)** |
| A,D | EP-A-0 352 182 (COMMISSARIAT A L'ENERGIE ATOMIQUE)<br>* Page 2, ligne 4 - page 3, ligne 19 *<br>--- | 1,6 | A 61 K<br>G 01 N<br>G 01 R |
| A | US-A-4 593 248 (J.S. HYDE et al.)<br>* Colonne 2, ligne 46 - colonne 3, ligne 31 *<br>--- -/- | 1 | |

Le présent rapport a été établi pour toutes les revendications

| Lieu de la recherche | Date d'achèvement de la recherche | Examinateur |
|---|---|---|
| LA HAYE | 24-09-1991 | HORAK G.I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Page 2

Office européen des brevets

# RAPPORT DE RECHERCHE EUROPEENNE

Numero de la demande

EP 91 40 1887

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EUROPHYSICS LETTERS, vol. 8, no. 3, 1 février 1989, pages 275-280, Strasbourg, FR; P. TUREK et al.: "Magnetic properties of the lithium phthalocyanine pi-radical. Role of dioxygen" * En entier * ----- | 1,6 | |
| | | | DOMAINES TECHNIQUES RECHERCHES (Int. Cl.5) |
| | | | |

Le présent rapport a été établi pour toutes les revendications

| Lieu de la recherche | Date d'achèvement de la recherche | Examinateur |
|---|---|---|
| LA HAYE | 24-09-1991 | HORAK G.I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)